Europäisches Patentamt

European Patent Office

Office européen des brevets

(11) Publication number: **0 365 198**
**A2**

## EUROPEAN PATENT APPLICATION

(21) Application number: **89310344.0**

(22) Date of filing: **10.10.89**

(51) Int. Cl.⁵: **A23K 1/00 , A01K 67/033 , C05F 3/00**

(30) Priority: **15.10.88 GB 8824207**

(43) Date of publication of application:
**25.04.90 Bulletin 90/17**

(84) Designated Contracting States:
**AT BE CH DE ES FR GB GR IT LI LU NL SE**

(71) Applicant: **MAGGOT FARMS (YORKSHIRE) LTD.**
**Denbrook**
**Conisbrough Nr. Doncaster(GB)**

(72) Inventor: **Savage, Donald Charles Stanley**
**The Manor House Northgate**
**Tickhill Nr. Doncaster(GB)**

(74) Representative: **Long, Edward Anthony et al**
**Hulse & Co. Cavendish Buildings West Street**
**Sheffield S1 1ZZ(GB)**

(54) **Feed material etc.**

(57) A method of producing a feed material for feeding maggots comprises sterilising and/or pasteurising by boiling and/or cooking a protein base material for a prescribed length of time, and within a prescribed temperature range, and introducing into the base material one or more of a sterilised and/or pasteurised additive material. Apparatus for carrying out this method comprises a sterilising and/or pasteurising vessel (1), an emulsifier (13) and a mixing vessel (19). The invention also includes a feed material produced by the method and apparatus. Another aspect of the invention relates to the processing of maggot farm residue comprising separating grown maggots from their feed bed, drying the separated material, crushing the separated material and introducing any additive, while apparatus for carrying this process comprises a riddle (33), a drier (30), various conveyor means (31, 32, 35, 37, 40), a mill (36), and preferably a bagging plant (41) and store (43).

## FEED MATERIAL ETC.

This invention relates to a method of producing a feed material for feeding maggots; to an apparatus for carrying out the method; to a material produced by the method; a production method for an organic agricultural material; to an apparatus for carrying out the method; and to the organic agricultural material produced by the method.

Conventionally, maggot farms have produced maggots for anglers or fishermen by providing a maggot feed material in the form of raw animal flesh such as beef, offal, poultry, fish etc., and a suitable environment to encourage rapid and satisfactory maggot growth. Such feed materials have not been free from botulinus and/or salmonella bacteria and indeed in some animal species, these organisms are endemic. It follows that maggots reared on such feed materials are likewise contaminated with botulinus and/or salmonella bacteria.

The basic object of the present invention is to provide a method and apparatus of producing a sterile and/or pasteurised feed material for maggots, whereby maggots free of botulinus and/or salmonella bacteria may be reared; to the feed material produced by the method; and to a method and apparatus for producing an organic agricultural product, and to the organic agricultural product produced by the method.

According to a first aspect of the present invention, there is provided a method of producing a feed material for feeding maggots comprising:-

(a) sterilising and/or pasteurising by boiling and/or cooking a protein base material for a prescribed length of time, and within a prescribed temperature range, such that the protein is sterilised and/or pasteurised yet remains as a slurry; and

(b) introducing into the base material one or more of a sterilised and/or pasteurised additive material such as: fish meal, meat meal, meat and bone meal, feather meal, blood meal, soya, greaves, sugar and molasses, to produce a final feed material being an admixture of sterilised and/or pasteurised protein slurry and sterilised and/or pasteurised additive materials.

The base material protein may be one or more of animal and/or fish flesh, blood, soya etc., and the additives may be introduced either before sterilising and/or pasteurising or after sterilising and/or pasteurising.

It may be that the nature of some of the protein start materials produces a slurry having a relatively coarse particle size whereas, if the feed material is for maggots, a relatively fine particle slurry is desirable. In this circumstance, it is preferred to effect - either before or after the admixture - the breaking down of the coarse particle slurry into a fine particle slurry of paste or jelly-like consistency for example in an em ulsifying or homogenising stage.

Furthermore, as a quality check, samples of the admixture may be taken from time to time to establish that the admixture is indeed sterile and/or pasteurised, as it is not impossible for supposedly sterile and/or pasteurised additives themselves to be contaminated. In the event of such contamination, the admixture is in accordance with another preferred feature of the first aspect of the invention, re-sterilised and/or re-pasteurised by boiling and/or cooking for a sufficient length of time and within a prescribed temperature range.

The additive materials are of course per se known proprietary products. However, in their as sold form they are inedible to maggots and consequently the mixing with the previously sterilised and/or pasteurised protein reconstitutes the additives to render them edible to maggots.

Clearly, the boiling/cooking time, and temperature range, either of the initial protein base material or as a result of any re-sterilisation or re-pasteurisation that may be necessary, may be reduced, in accordance with a preferred proposal, by operating at super atmospheric pressure.

According to a second aspect of the invention, there is provided apparatus for carrying out the above defined method comprising:-

(a) a sterilising and/or pasteurising vessel adapted to receive an unsterilised and/or unpasteurised protein base material and subsequently to boil and/or cook a protein base material;

(b) means to introduce sterilised and/or pasteurised additives to the base material; and

(c) means to remove the admixture from the vessel.

It will be appreciated that if the additives are introduced at an early stage, the protein base material will not be sterilised and/or pasteurised, while if introduction is delayed, the protein base material may well be sterilised and/or pasteurised.

Thus, both the sterilising and/or pasteurising and the additive introduction may be effected in one and the same vessel, but if more continuous production is required, it is preferred to provide a separate mixing vessel and means to transfer the sterilised and/or pasteurised base material as a slurry from the sterilising and/or pasteurising vessel to the mixing vessel.

If the sterilised and/or pasteurised base material is oversized, then in accordance with another preferred feature the base material is transferred firstly to size-reducing vessel, such as an emulsifier, homogenizer etc., to effect particle breakdown to a desired size e.g., a paste or jelly-like consistency, and is thereafter transferred to the mixing vessel.

Preferably, the sterilising and/or pasteurising vessel is a pressure vessel and is therefore operable at super atmospheric pressure to reduce the sterilising and/or pasteurising time.

Preferably, the sterilising and/or pasteurising vessel is jacketed, to receive a heating medium.

The mixing vessel may also serve as a storage or holding tank and is preferably also a pressure vessel so that, should any re-sterilisation or re-pasteurisation prove necessary, this may be effected in the mixing vessel. The latter is conveniently jacketed to receive a heating medium.

The heating medium - preferably steam - is conveniently produced at a boiler, with a feed line with a central valve(s) to the sterilising and/or pasteurising vessel, and a return line from that vessel. Similar feed and return lines may be provided if a re-sterilising and/or re-pasteurising capability is required for the mixing vessel.

At the close down of operations, and with the equipment empty of materials, the apparatus itself may be sterilised by passing steam therethrough.

Transfer of material between the sterilising and/or pasteurising vessel and the sizing vessel and the emulsifier and the mixing vessel is preferably via pipelines with suitable pumps and control valves. Thus, an outlet pipeline from the sterilising and/or pasteurising vessel may be provided with a control valve and downstream thereof a pump (preferably electrically powered) the outlet pipeline progressing to an inlet pipeline of the sizing vessel, which inlet pipeline is provided with a control valve. Similarly, an outlet pipeline from the sizing vessel is provided with a control valve and downstream thereof with a pump, the outlet pipeline progressing to an inlet pipeline of the mixing vessel, similarly an outlet pipeline of the mixing vessel is provided with a control valve and downstream thereof, a pump. The latter may supply the feed material to a mobile feeding unit if the apparatus is located at a maggot farm. It follows that at the close-down of operations, the pipelines and pumps may be similarly sterilised by passing steam therethrough.

To introduce the protein base materials into the sterilising and/or pasteurising vessel, the latter may be provided with a hopper or funnel, with a control valve at its lower end, to control the rate of fall of protein base material from the hopper or funnel into the sterilising and/or pasteurising vessel.

According to a third aspect of the invention there is provided a feed material for maggots, produced by the method and apparatus defined above.

In practice, maggots are fed in a loose bed of a mix of feed material (produced in accordance with the first aspect of the invention), and sawdust etc, and after attaining a desired size and weight the maggots are separated from the bed. The bed residue therefor consists of a mix of sawdust etc., protein matrial consumed and passed through the maggot larvae and usually, a small quantity of unconsumed feed material, and this can form the basis of a completely organic agricultural material which, when suitably processed, is suitable for the filling of grow bags or for direct application to agricultural land.

Therefore, in accordance with a fourth aspect of the invention, there is provided a method of processing maggot farm residue comprising unconsumed maggot feed material produced in accordance with the first aspect of the invention, to produce a completely organic agricultural material, comprising,

(a) separating grown maggots from their feed bed;

(b) drying the separated material to remove any odour;

(c) drying any organic additive required to be added to the separated and dried material;

(d) milling the separated dried material, or the mix of separated dried material and organic additive, to obtain a maximum particle size; and

(e) introducing any required mineral additives, in powder form, to produce the agricultural material.

The organic additive may for example be chicken manure, while the mineral additive may be an alkali material(s) such as crushed limestone, to produce a desired pH value. The mineral is conveniently introduced by injection.

According to a fifth aspect of the invention, there is provided apparatus for carrying out the above defined method comprising:

(a) a riddle to effect maggot/feed bed separation;

(b) a dryer;

(c) a conveyor between the riddle and the dryer;

(d) a mill; and

(e) a conveyor between the dryer and the mill.

For the addition of any organic additive, a hopper is provided to receive the additive, with a conveyor between the hopper and the dryer.

For the introduction of any mineral, a blender is provided after the mill, with a conveyor between the mill and the blender.

If the agricultural material is required as filling for grow bags, then a bagging plant is provided with a conveyor between the mill and the bagging plant or between any blender and the bagging plant. The filled bags may then be loaded onto pallets for transport or storage.

Conveniently, the apparatus in accordance with the fifth aspect may be integrated with the apparatus in accordance with the second aspect - for instance the source that provides the heating medium for the boiling and/or cooking vessel, and possibly for the mixing vessel, conveniently also provides the heating medium for the dryer. If, in accordance with the preferred proposal, the heating medium is steam provided by a boiler, a separate feed line, with a suitable control valve(s), extends between the boiler and the dryer.

According to a sixth aspect of the invention, there is provided an agricultural material produced by the method and apparatus defined above.

The various aspects of the invention, will now be described in greater detail, by way of example, with reference to the accompanying diagrammatic drawings.

In the drawings is illustrated a sterilising and/or pasteurising vessel 1 having an external jacket 2 by which the vessel 1 is heated with steam supplied from a boiler 3 via distribution lines 4 and control valves 5. Above the vessel 1 is an infeed pipe 6 to supply unsterilised and/or unpasteurised protein base material to the vessel 1 from a supply hopper 7 having a control valve 8.

After heating and/or pasteurising of the protein base material in the vessel 1, the base material is discharged via an outlet pipe 9 connected to the base of the vessel 1 and housing a control valve 10, the pipe 9 leading to a pump 11 from which extends a delivery pipe 12 to an emulsifier 13 in which the sterilised and/or pasteurised base material is sized to a paste like consistency, with a control valve 14 controlling inlet to the emulsifier, and a similar control valve 15 located in an outlet pipe 16 to control outlet of the emulsified base material from the emulsifier 13, the outlet pipe 16 leading to a pump 17 from which extends a delivery pipe 18 to a mixing vessel 19 also having a jacket 20 to receive steam via a line 4 and control valve 5. The mixing vessel 19 is also provided with an inlet pipe 21 for the introduction of sterilised and/or pasteurised additives supplied from a hopper 22 having a control valve 23. When it is required to remove the admixture of base material and additive from the mixing vessel 19, which can also serve as a storage tank should immediate removal of the contents of the vessel not be required, a valve 24 in a discharge pipe 25 is opened so that the content s are supplied to a pump 26 having a delivery line 27 to a mobil e feeding unit for example, while live steam is returned from the mixing vessel 19 to the sterilising and/or pasteurising vessel 1 via a line 28 with a control valve 29.

Thus, the elements 1-29 constitute basically the apparatus in accordance with the second aspect of the invention, for carrying out the method in accordance with the first aspect of the invention.

Consequently, there will now be described apparatus in accordance with the fifth aspect of the invention, for carrying out the method in accordance with the fourth aspect of the invention.

From the boiler 3 also extends a steam supply line 4A to a drier 30 having associated conveyors 31 and 32 extending respectively from a riddle 33 and a hopper 34. From the riddle 33 the drier 30 is supplied with separated material, being basically a mix of unconsumed feed material, sawdust etc., and maggot waste, and from the hopper 34 the drier 30 is supplied with organic material to be added to the separated material. After drying, the mix of separated material and organic additive is passed by a conveyor 35 to a mill 36 in which the mix is crushed, with the mix then being passed by a conveyor 37 to a blender 38 associated with injection equipment 39 whereby mineral additives, such as crushed limestone are injected to the mix, with a conveyor 40 extending to a bagging plant 41, and with the bagged mix passed by a conveyor 42 (or by a pallet or truck) to a store 43.

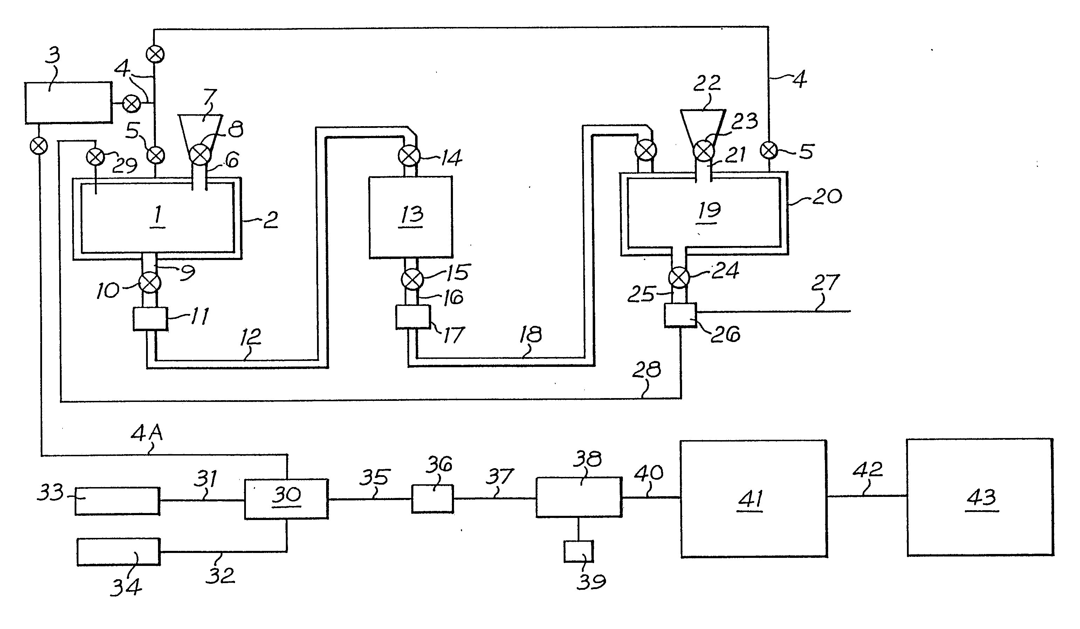

## Claims

1. A method of producing a feed material for feeding maggots comprising:-

   (a) sterilising and/or pasteurising by boiling and/or cooking a protein base material for a prescribed length of time, and within a prescribed temperature range, such that the protein is sterilised and/or pasteurised yet remains as a slurry; and

   (b) introducing into the base material one or more of a sterilised and/or pasteurised additive material such as: fish meal, meat meal, meat and bone meal, feather meal, blood meal, soya, greaves sugar, molasses to produce a final feed material being an admixture of sterilised and/or pasteurised protein slurry and sterilised and/or pasteurised additive materials.

2. A method as claimed in Claim 1, in which the base material protein is one or more of animal and/or fish flesh, blood, soya.

3. A method as claimed in Claim 1 or Claim 2, in which the additives are introduced before or after sterilising and/or pasteurising.

4. A method as claimed in any preceding Claim, wherein the slurry, either before or after the admixture, is broken down from a coarse particle slurry into a fine particle slurry of paste or jelly-like consistency for example in an emulsifying or homogenising stage.

5. Apparatus for carrying out the above defined method comprising:-

   (a) a sterilising and/or pasteurising vessel (1) adapted to receive an unsterilised and/or unpasteurised protein base material and subsequently to boil and/or cook a protein base material;

(b) means (21 - 23) to introduce sterilised and/or pasteurised additives to the base material; and

(c) means (24 - 27) to remove the admixture from the vessel.

6. Apparatus as claimed in Claim 5, comprising a separate mixing vessel (19) and means (9 - 14, 15 - 18) to transfer the sterilised and/or pasteurised base material as a slurry from the sterilising and/or pasteurising vessel (1) to the mixing vessel (19).

7. Apparatus as claimed in Claim 5 or Claim 6 comprising a size-reducing vessel such as an emulsifier, homogenizer etc., to effect particle breakdown to a desired size e.g., a paste or jelly-like consistency of the sterilised and/or pasteurised base material.

8. Apparatus as claimed in any one of Claims 5 to 7, wherein heating medium in the form of steam is produced at a boiler (3), with a feed line with a control valve(s) (5) to the sterilising and/or pasteurising vessel (1), and a return line from that vessel.

9. Apparatus as claimed in any one of Claims 5 to 8, wherein pipelines (9, 12, 16, 18, 25) with suitable pumps (11, 17, 26) and control valves (10, 15, 24) are provided for introduction and/or transfer and/or removal of the base material and/or the additive material and/or the feed material to, between, or from, various elements of the apparatus.

10. A method of processing maggot farm residue comprising unconsumed maggot feed material produced in accordance with the method defined in any one of Claims 1 to 4 to produce a completely organic agricultural material, comprising,

(a) separating grown maggots from their feed bed;

(b) drying the separated material to remove any odour;

(c) drying any organic additive required to be added to the separated and dried material;

(d) crushing the separated dried material, or the mix of separated dried material and organic additive, to obtain a maximum particle size; and

(e) introducing any required mineral additives, in powder form, to produce the agricultural material.

11. A method as claimed in Claim 10, wherein the organic additive is chicken manure.

12. A method as claimed in Claim 10 or Claim 11, wherein the mineral additive is an alkali material(s) such as crushed limestone, to produce a desired pH value.

13. Apparatus for carrying out the method of any one of Claims 10 to 12 comprising: (a) a riddle (33) to effect maggot/feed bed separation;

(b) a dryer (30);

(c) a conveyor (31) between the riddle (33) and the dryer (30);

(d) a mill (36); and

(e) a conveyor (35) between the dryer (30) and the mill (36).

14. Apparatus as claimed in Claim 13, wherein for the addition of any organic additive, a hopper (34) is provided to receive the additive, with a conveyor (32) between the hopper (34) and the dryer (30), while for the introduction of any mineral, a blender (38) is provided after the mill (36), with a conveyor (37) between the mill (36) and the blender (38).

15. Apparatus as claimed in Claim 13 or 14, in combination with the apparatus defined in any one of Claims 5 to 9.

16. A feed material for maggots produced by the method of any one of Claims 1 to 4, and/or by the apparatus of any one of Claims 5 to 9.

17. An agricultural material produced by the method of any one of Claims 10 to 12, and/or the method and apparatus of any one of Claims 13 to 15.